# EUROPEAN PATENT APPLICATION

(11) **EP 2 264 054 A1**
(43) Date of publication of application: **22.12.2010**
(21) Application number: 09162935.2
(22) Date of filing: 17.06.2009
(51) Int. Cl.: C07K 14/415

(54) **Cloning, yeast expression, purification and biological activity of a truncated form of the soybean 7S globulin alfa ' subunit involved in Hep G2 cell cholesterol homeostasis**

(71) Applicant: Indena S.p.A., 20139 Milano (IT)
(72) Inventor: Morazzoni, Paolo, 20139 Milan (IT); Riva, Antonella, 20139 Milan (IT); Ponzone, Cesare, 20139 Milan (IT); Berlanda, Davide, 20139 Milan (IT); Duranti, Marcello, 20133 Milan (IT); Consonni, Alessandro, 20133 Milan (IT)
(74) Representative: Minoja, Fabrizio

(57) **Abstract**

A truncated form of α' chain (tα'), the soybean 7S globulin, active in controlling the cholesterol and triglyceride homeostasis in *in vitro* and *in vivo* models, was cloned and expressed in the yeast *Pichia pastoris.* The recombinant polypeptide spanned 216 amino acid residues from the N-terminal side and included the N-terminal extension region of the soybean subunit. The tα' polypeptide was purified by conventional biochemical techniques and its potential to modulate the activity of the LDL-receptor was evaluated in a human hepatoma cell line (Hep G2) by monitoring the uptake and degradation of labeled LDL. The LDL uptake (+ 192%) and degradation (+ 143%) by cells tested at the highest tα' dose (8 µM) were similar to those found in cells incubated with 1 µM simvastatin, a potent inhibitor of cholesterol biosynthesis. The cell response to tα' was found to be dose-dependent.

## Description

The present invention refers to a truncated recombinant α' subunit of the soybean 7S globulin comprising the N-terminal side of the natural α' subunit, to a process for its preparation and to compositions containing it as an active ingredient useful for controlling the cholesterol and triglyceride homeostasis.

### BACKGROUND OF THE INVENTION

The role of dietary soybean proteins in the control of lipidemic levels of hypercholesterolemic patients is a widely accepted issue [1]. In previous studies [2-4], the direct involvement of one subunit of the soybean 7S globulin, the α' subunit, in the up-regulation of the LDL-receptor was demonstrated in *in vitro* and *in vivo* systems, suggesting that biologically active (poly)peptides, capable of modulating cholesterol homeostasis, are likely to be produced by cell enzyme processing.

The native 7S globulin is composed of three randomly assorted polypeptide chains, the α', α and β subunits [5], encoded by different genes. The mature α' (Accession N. P11827 UniProtKB/Swiss-Prot database) and chains (Accession N. P13916 UniProtKB/Swiss-Prot database) share an extended N-terminal region of about 145 amino acid residues which is missing in the β subunit (Accession N. P25974 UniProtKB/Swiss-Prot database). Based on the peculiar amino acid sequence of the α' extension region, this subunit was purified by metal affinity chromatography and orally administered to hypercholesterolemic rats, thus allowing to show both its plasma lipid-lowering properties and up-regulation of liver β-VLDL receptors [4]. On the other hand, since the molecular weight of the α' subunit is around 71 kDa [6], it seems unlikely that it may cross *in vivo* the intestinal barrier with no modification.

For this reason, our research has been directed to seeking the amino acid sequence/s of α' subunit responsible for the pharmacological effect. Since the core regions of the three subunits have more similar amino acid sequences, it is conceivable that the biological activity should reside in one or more (poly)peptides of the extension region. In principle, the localized but significant amino acid differences between the extension regions of the α' and α chains would limit the number of peptides responsible for the biological activity.

From these previous statements, the first strategy pursued was to test the effect of both polypeptides in Hep G2 cells, obtained from the *in vitro* digestion (pepsin/trypsin) of Croksoy^{R}70, an isoflavone-free soybean concentrate routinely employed in the dietary treatment of hypercholesterolemic patients [7-8], and synthetic peptides, corresponding to specific amino acid sequences that differed between the 7S soybean globulin subunits, on the LDL-receptor (LDL-R) modulation. The results obtained in these studies pointed out that a marked LDL-R up-regulation could be induced in HepG2 cells exposed to enzyme digestion products of Croksoy^{R}70 with MW ranging from 3,000 to 20,000 Da, as well as to a small synthetic peptide (2,271 Da) from 7S soybean globulin added to cells at a concentration of 10-⁴M [9]. The study obtained with small peptides is still currently under investigation and have not been conclusive so far [10].

The cholesterol and triglyceride lowering capacity of soybean proteins is a consolidated issue. The soybean protein diet is currently the most potent dietary tool for treating hypercholesterolemic patients, thus providing a unique opportunity for the management of adults and very young subjects. Moreover, it is clearly established that the plasma cholesterol reduction is greater in patients having a high baseline degree of cholesterolemia [14].

The hypothesis that proteins *per se* reduce blood cholesterol arose from experimental studies indicating that a shift from animal to plant proteins in the diet activates the LDL receptor system in the liver of laboratory animals [15], as well as in circulating lymphomonocytes of hypercholesterolemic patients [16]. To identify the soybean protein components responsible for the cholesterol lowering effect, *in vitro* studies were carried out with a human hepatoma cell line that is highly sensitive to factors regulating LDL-receptor expression and cholesterol biosynthesis/breakdown. The purified α' subunit from the 7S soybean globulin was found to up-regulate LDL-receptors in Hep G2 cells [3] and this finding was confirmed in cholesterol-fed rats [4]. Although these data support the hypothesis that the protein moiety is responsible for the observed biological effect, arguments may be raised to α' chain *in vivo* biological fate, since peptides and amino acids are normally produced by the action of gastric and/or intestinal proteolytic enzymes. However, an increasing number of animal and plant (poly)peptides is being claimed to play relevant regulatory functions, often attributed to anti-oxidant, anti-proliferative and anti-inflammatory effects [17]. As far as soybean is concerned, experimental evidence clearly indicates the possibility that peptides and even small compact proteins, such as the Bowman-Birk inhibitor, may be adsorbed [18], thus eliciting a number of effects, including anticancer, anti-inflammatory, radio-protective ones [19]. Also genetically modified soybean (poly)peptides have been shown to trigger biological responses, such as hypotensive effects [20]. Recently, a LDL-R transcription stimulating peptide (FVVNATSN), deriving from the 7S globulin β chain, has been identified from a soybean hydrolysate prepared by a protease from *Bacillus amyloliquefaciens* and then by chemical synthesis [21]. In this case, an increased LDL-R transcription (+148%) was detected in Hep G2 cells exposed to the peptide at a concentration of 100 µM. Other peptides arising from the 11 S globulin have been shown to exert similar but lower activity [21].

It would be desirable to make available shorter polypeptides maintaining or even improving the biological properties of the full length protein.

### DESCRIPTION OF THE INVENTION

It has now been found that a truncated α' Subunit of Soybean 7S Globulin comprising the N-terminal side thereof has advantageous biological activity and proved to be even more effective on LDL uptake and degradation than the full size α' chain.

The invention provides therefore said truncated α' Subunit, which will be hereinafter referred to as tα', as well as a process for its preparation by cloning, yeast expression and purification of the recombinant polypeptide containing d the N-terminal extension region of the soybean α' subunit

To this purpose the heterologous expression of a truncated form of the α' chain was undertaken. The objective was achieved in secretion-competent yeast cells of yeast *Pichia pastoris.* The recombinant polypeptide was purified and its biological activity assessed in HepG2 cells. By the use of this biotechnological approach, adequate amounts of the recombinant polypeptide could be obtained to be tested in *in vitro* trials and also in *in vivo* experiments.

### DESCRIPTION OF THE FIGURES

Fig. 1. Overview of the pPICZαB-tα' construct. Expression of tα' is driven by the AOX (Alcohol OXidase) methanol-inducible promoter (5'AOX1); the α-Mating Factor (α-MF) promotes secretion of the recombinant protein to the medium; AOXI TT: AOX transcription termination region. *Sh ble* gene confers resistance to zeocin; pUC Ori: origin of replication for high number plasmid copy in *E. coli.* The other abbreviations refer to the cleavage positions of restriction enzymes; bp: base pair.
Fig. 2 Sequence alignment of the recombinant polypeptide (tα') (SEQ ID 1) and wild type soybean α' subunit (SEQ ID 2). Stars indicate identical amino acid residues in the two sequences.
Fig. 3 SDS-PAGE under reducing conditions of *Pichia pastoris* culture media (A) and tα' purification steps (B).
   A: Lane 1: X33-pPICZαB (empty construct) induced with 1% of methanol; Lane 2: X33-pPICZαB-tα' induced with 1% of methanol.
   B: Lane 3: acetone powder; Lane 4: DEAE-cellulose unbound fraction; Lane 5: DEAE-cellulose 150 mM NaCl eluted fraction; Lane 6: DEAE-cellulose 250 mM NaCl eluted fraction.

### DETAILED DESCRIPTION OF THE INVENTION

The invention will now be described in detail in the following experimental section.

### Materials and Methods

***Yeast, bacterial strains and chemicals.** Pichia pastoris X33* (WT) strain (Invitrogen, San Diego, CA) was used for yeast expression. The bacterial strain utilized for genetic manipulations was *E. coli* XL1-Blue (Invitrogen, San Diego, CA). Restriction enzymes Pst I and Xba I were purchased from Roche (Indianapolis, IN), Sac I from Fermentas (Ontario, Canada) Taq DNA polymerase was purchased from Invitrogen (San Diego, CA). Zeocin was purchased from Invivogen (San Diego, CA). The oligonucleotides for PCR were obtained from Primm (Milano, Italy). Peptone, tryptone, yeast extract and agar were purchased from Becton Dickinson and Company (Sparks, MD).

Other chemicals were reagent grade from Sigma (St. Luis, MO).

***Media and growth conditions.** P. pastoris X33* strain was cultured in YPD (Yeast Peptone Dextrose) complete medium (2% peptone, 1% yeast extract, 2% glucose). The Mut⁺ transformants were selected in plates containing YPD, agar 1.5%, 100 mg/mL zeocin. All yeast cultures were maintained at 30°C. For protein production, selected Zeo^{r}-Mut⁺ transformants were cultured to an optical density of 5 at 600 nm in YPS (Yeast Peptone Sorbitol) medium (2% peptone, 1% yeast extract, 2% sorbitol). Then methanol was added to 1% final concentration. All bacterial transformants were selected in plates of low-salt LB (Luria-Bertani) medium containing zeocin (1% tryptone, 0.5% yeast extract, 0.5% sodium chloride, 1.5% agar, 25 mg/mL zeocin). All bacterial cultures were maintained at 37°C.

***Construction of t***α***' gene expression vector.*** The tα' gene was amplified by PCR on an expression *E*. *coli* plasmid DNA template (pT7-7) containing the sequence of interest (tα'). The oligonucleotide PIC-1 (5'-GAAGGAGATATACATG**CTGCAG**TGGAGGAAG-3') (SEQ ID 3) was designed to generate PstI restriction site at the 5' end of tα' gene. This mutation resulted in an alanine residue insertion in the N-terminal position of tα' A second oligonucleotide PIC-2 (5'-CGATGC**TCTAGA**TCATCAGTTAAGGATAACGATG-3') (SEQ ID 4)was designed to generate XbaI restriction site at the 3' end of tα' gene. Both oligonucleotides were dissolved in mQ sterile water. The PCR reaction mixture consisted of 0.5 mM primers, 0.8 mM dNTPs (Eppendorf, Hamburg, Germany), 30 ng template (pT7-7/tα'), 2.5 U Taq DNA Polymerase, PCR buffer (final composition: 50 mM KCl, 1.5 mM MgCl₂, 20 mM Tris-Cl, pH 8.4 and mQ sterile water to a final volume of 25 mL). PCR amplification was carried out on a Perkin Elmer Geneamp PCR System 2400 thermocycler (Perkin Elmer Corp., Wellesley, MA) using the following conditions: start at 97°C for 6 min; 30 cycles at 97°C for 1 min, 50°C for 1.5 min, 72°C for 1.5 min; final extension 72°C for 10 min and maintained at 4°C. The PCR 650 bp product was cloned into pPICZaB vector resulting in the pPICZaB-tα' construct and transformed in XL1-Blue *E. coli* cells. Positives clones were selected on semisolid LB media containing tetracycline and zeocin. One of these clones was sequenced by Primm (Milano, Italy) to ensure that no mutation occurred in pPICZaB-tα' construct sequence. Thirty five mg pPICZaB-tα' construct and 30 mg expression vector pPICZaB (negative control) were linearized by digestion with the restriction enzyme SacI and then purified.

***Transformation of pPICZaB-t***α***' in P. pastoris genome**.* Wild type (wt) yeast cells were transformed by electroporation with 35 mg linearized pPICZaB-tα' construct and 30 mg linearized pPICZaB on an Eppendorf (Hamburg, Germany) electroporator 2510 apparatus set at 1.5 kV. Transformants were first selected by plating on YPD plates containing 100 mg/mL of zeocin. In order to verify the integration of our construct into transformed *P. pastoris* genome, the Dneasy Plant Mini Kit (QIAGEN, Hilden, Germany) was used to extract genomic DNA. Genomic DNA was used as template to verify the insertion of the construct at the alcohol oxidase promoter (AOX1) site by PCR using 5'AOX1 (5'-GACTGGTTCCAATTGACAAGC-3') and 3'AOX1 primers (5'-GCAAATGGCATTCTGACATCC-3') (SEQ ID 5). The PCR reaction mixture consisted of 0.5 mM primers, 0.25 mM dNTPs, 100 ng for genomic template and 30 ng for plasmidial template, 2.5 U Taq DNA Polymerase, PCR buffer (final composition as above). PCR reaction was performed using the following conditions: start at 94°C for 10 min; 30 cycles at 94°C for 40 s, 60°C for 40 s, 72°C for 20 s; final extension 72°C for 10 min and maintained at 4°C.

***Expression and purification of t***α***'.*** Eighteen Zeo⁺ transformants and one transformant containing pPICZaB expression vector (negative control) were grown in 50 mL of YPS medium at 30°C in a shaking (180 rpm) incubator to an OD₆₀₀ = 5. The inducing phase was triggered by adding methanol to 1% final concentration and prolonged for 24 hours. Aliquots of the supernatants were examined for expression of tα' by SDS-PAGE. The clone transformant with the highest tα' expression was selected for massive production of the recombinant protein and grown under the same conditions as above. About 1 L culture was centrifuged at 11,300 x g and 24°C for 15 min with a J2-21 M/E, Beckman Instrument, Palo Alto, CA. The proteins contained in the supernatant were precipitated by 70% ammonium sulfate and centrifuged at 11,300 x g at 4°C for 30 min. The pellet was dissolved in 50 mL mQ water and precipitated by 100% acetone (1/1 v/v) pre-freezed at -30°C. The solution was centrifuged for 1 hour as above. The pellet was dissolved in 25 mL 50 mM Tris-HCl, pH 7.40 and loaded on a DEAE-cellulose column (2.5 x 10 cm, Whatman, Maidstone, UK) equilibrated with the same buffer. The elution of retained proteins was carried out with the same buffer containing 0.15 and 0.25 M NaCl respectively. The fraction eluted with 0.25 M NaCl displayed the greatest content of tα'.

***Electrophoretic techniques.*** SDS-PAGE under reducing conditions (2% β-mercaptoethanol) was carried out on 12% polyacrylamide gels, according to ref. 11, using a mini-Protean II cell (Bio-Rad). The gels were stained with Coomassie Blue.

***Cell cultures.*** The established human hepatoma cell line (HepG2) was obtained from American Type Culture Collection (Rockville, MD). Eagle's minimum essential medium (MEM), fetal calf serum, trypsin-EDTA (1x), penicillin (10⁵ U/L), streptomycin (100 g/L), tricine buffer (1 mmol/L, pH 7.4) and non-essential amino acid solutions (100x) were from GIBCO (Madison, WI). Petri dishes were from COSTAR (Cambridge, MA). Filters were from Millipore (Bedford, MA). The Protein Coomassie Plus Protein Assay kit was purchased from Pierce (Rockford, IL, USA). ¹²⁵Iodine, carrier free, in 100 mmol/L NaOH, was from Perkin Elmer Life Sciences (Boston, MA). Sephadex G25 columns (PD10) were from Pharmacia Biotech (Uppsala, Sweden). LDH and MTT kit were from Sigma Diagnostics (Milano-Italy). All other chemicals were of analytical grade from Merck (Darmstadt, Germany). Cells were grown in monolayers in 90 mm-diameter Petri dishes, and maintained at 37°C in a humidified atmosphere of 95% air, 5% CO₂ in MEM supplemented with 10% fetal calf serum (FCS), non essential amino acid solution (1%, v/v), penicillin (10⁵ U/L), streptomycin (0.1 g/L), tricine buffer (20 mmol/L, pH 7.4), NaHCO₃ (24 mmol/L) and sodium pyruvate (0.11 g/L). For experiments designed to evaluate the LDL receptor modulation, cells were seeded in 35 mm plastic dishes (3-5 x 10⁵ cells) and used just before reaching confluence. In all cell culture experiments, the medium was changed every 2-3 days. In order to assess cell viability, culture media from cells exposed to tα' at different concentrations were tested by methyltetrazolium salts (MTT) assay, essentially as described in ref. 9. Cell enzyme leakage was determined by measuring lactate dehydrogenase (LDH) activity, using a kinetic (LDH/LD) diagnostic kit (Sigma Diagnostics). LDL (1.019≤ d ≤1.063 g/L) were isolated by sequential preparative ultracentrifugation [12] from the plasma of clinically healthy normolipidemic volunteers. Lipoproteins were labeled according to the method of McFarlane as modified by Bilheimer et al. [13], and previously described [3]. ¹²⁵I-LDL were sterilized by filtration (Millipore filters, 0.45 µm pore size) and stored at 4°C until use. Human lipoprotein deficient serum (LPDS) was prepared as previously described [9].

***Uptake and degradation of ¹²⁵I-LDL.*** Monolayers of cells were preincubated at 37°C for 24 h in MEM supplemented with 5g/100g LPDS to up-regulate the LDL-receptors [2], in the presence/absence of tα' at different concentrations listed in Table or 3.5 µmol/L α' purified subunit or 1.0 µmol/L simvastatin. A fixed concentration (7.5 mg/L) of ¹²⁵I-LDL was then added to the medium and the incubation continued for a further 5 h at 37°C. Specific uptake (binding + internalization) and degradation of ^{l25}I-LDL were evaluated as previously reported [2].
***Statistical analyses.*** Differences in cell uptake and degradation of LDL after cell incubation with tα' at different concentrations were determined by ANOVA followed by Dunnett's test. Values are expressed as means ± SD; *P* values < 0.05 were considered as statistically significant.

### Results

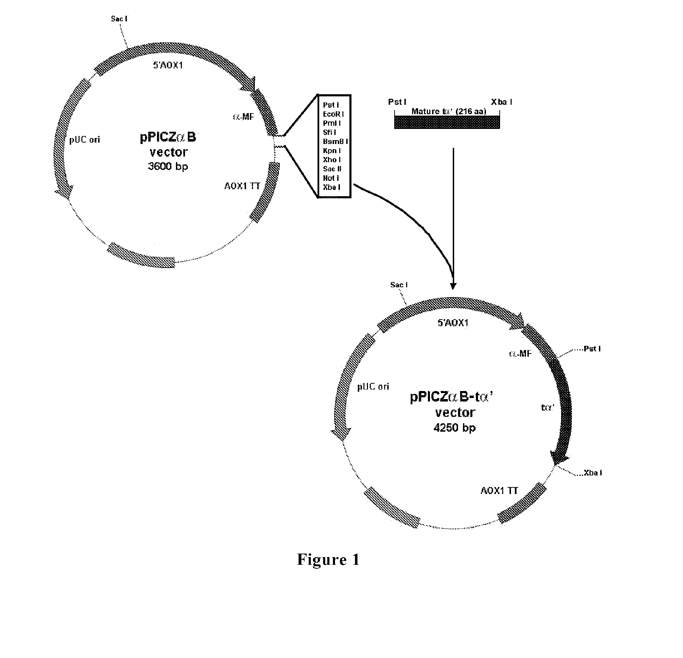

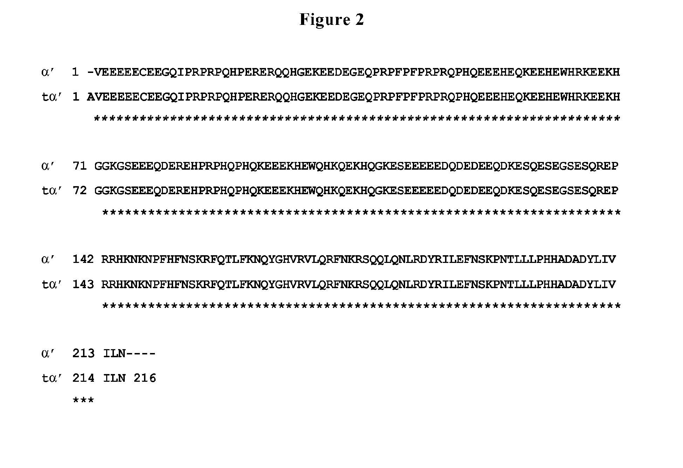

***Expression of t***α***' in Pichia pastoris.*** The structure of the plasmid used to transform *Pichia pastoris* cells is shown in **Figure 1****.** The sequence of the insert and its alignment with the α' subunit are shown in **Figure 2****.** As mentioned in the Materials and Methods, the only difference between the recombinant and wild type polypeptides consisted in the N-terminal first amino acid residue which, for technical reasons, was an alanine in the recombinant chain. The clone showing the greatest production of recombinant polypeptide, as judged by SDS-PAGE analysis of the culture medium (not shown), was selected for massive production.

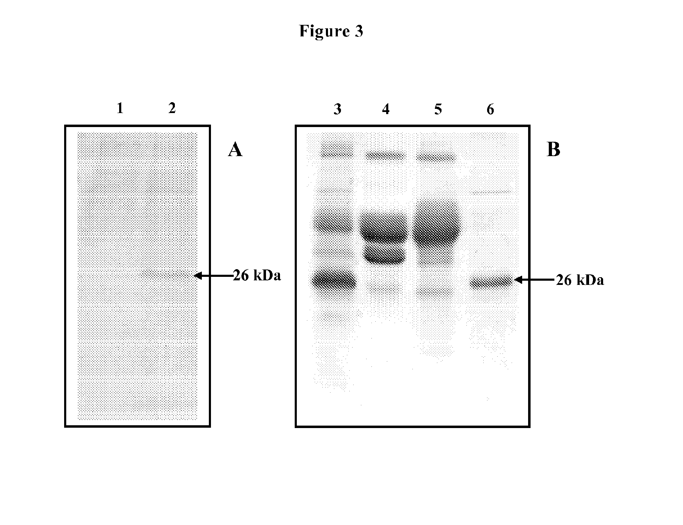

***Purification of t***α*'.* The purification of tα' was achieved by a combination of precipitation steps and chromatographic approaches. Samples from each step were collected and analyzed by SDS-PAGE **(****Fig. 3****).** The panel A of **Fig. 3** shows the electrophoretic patterns of the culture media corresponding to the yeast clone transformed with an empty and integrated construct, lanes 1 and 2 respectively. At it is shown, a faint band at 26 kDa was detected only in the positive clone, indicating the expression of the recombinant polypeptide. N-terminal sequence analysis of this band confirmed that this 26 kDa polypeptide corresponded to the tα' chain. The effect of the purification steps on the homogeneity of the identified polypeptide is shown in fig. 3, panel B. A relevant enrichment of the polypeptide was already achieved with the precipitation procedures, but the further chromatographic steps removed main contaminant proteins and allowed the recovery of the recombinant polypeptide in an almost homogenous form. The purity of this sample was judged to be suitable for the cell assays.

***Biological activity of t***α***'.*** The addition of the purified α' subunit, as a positive control, and its truncated α' form to HepG2 cells produced a significant rise in LDL receptor-mediated uptake and degradation compared to the untreated cells , as reported in the following Table.

**Table. Effect of α' and its truncated form (tα') on the LDL uptake and degradation by HepG2 cells^{1,2}**

| Sample type | Concentration³ | Uptake | | Degradation | |
|---|---|---|---|---|---|
| | µ*mol*/*L* | *ng¹²⁵I-LDL*/ *mg cell proteins* | % | *ng¹²⁵I-LDL*/ *mg cell protein* | *%* |
| LPDS⁴ | - | 90 ± 3.0 | 100 | 78 ± 2.3 | 100 |
| 7S α' subunit | 3.5 | 164 ± 8.1* | 182 | 138 ± 3.8* | 177 |
| tα' | 1.0 | 147 ± 9.5* | 163 | 154 ± 8.7* | 197 |
| | 2.0 | 182 ± 8.6* | 202 | 179 ± 7.5* | 229 |
| | 4.0 | 208 ± 10.3** | 231 | 187 ± 6.2** | 240 |
| | 8.0 | 263 ± 7.9** | 292 | 189 ± 6.5** | 243 |
| Simvastatin | 1.0 | 250 ± 11.2** | 278 | 195 ± 7.9** | 250 |

| | | | | | |
|---|---|---|---|---|---|
| ¹the data are means ± SD of 3 independent experiments, each performed in quadruplicate. **P*< 0.05 *vs* LPDS and **P<0.001 *vs* LPDS. ²Confluent monolayers of HepG2 cells were preincubated for 24 h at 37°C in minimum essential medium with 5% LPDS, in the presence or absence of different concentrations of recombinant polypeptide (tα') or purified α' subunit (7S α') or simvastatin. After the addition of ¹²⁵I-LDL (7.5 mg/L of medium), cells were incubated for an additional 5 h. ⁴LPDS, lipoprotein-deficient serum. | | | | | |

The LDL modulation was dose-dependent with tα' and greater than that recorded in cells incubated with the purified α' subunit. The result obtained with tα' at the highest concentration was similar to that of the positive control, simvastatin. At no concentration of tα' was there any evidence of cellular toxicity, as determined by the MTT and LDH assays (not shown).

It has been therefore found according to the invention that the amino acid sequence capable of inducing the biological response lies in the N-terminal extension domain of α' chain. Moreover, we found that the truncated polypeptide exerts its effects at concentrations in the order of magnitude as those of simvastatin, a potent hypolipidemic drug. This effect might at least partially be due to the *in vitro* interaction between the above fragment globulin and thioredoxin, a small multifunctional protein with a redox-active disulfide-dithiol in the conserved active site sequence Cys-Gly-Pro-Cys, as reported by the present inventors [3]. This finding might explain the longer lag phase of LDL oxidation induced by cupric oxide observed in rabbits fed cholesterol-rich diet containing soybean protein *versus* that found in rabbits fed the same diet but containing casein as protein source [22].

The data obtained are particularly interesting because they show for the first time that a truncated recombinant form of soybean 7S globulin α'chain is active in *in vitro* model at concentrations less than 10 µM, that are similar that those reported for simvastatin. Moreover, the use of a recombinant protein rules out any involvement of other protein and non-protein soybean components, including isoflavones, for which a lack of clear benefits and potential toxicity have been reported[23].

The invention provides functional foods and compositions with beneficial effects on various diseases, including hyperlipidemia and cardiovascular disease, to be used alone or in combination with drugs in lipid-lowering therapies, i.e. statins such simvastatin, pravastatin, fluvastatin, atorvastatin, lovastatin.

The compositions of the invention are prepared using conventional excipients and methods. The dosage of the recombinant polypeptide of the invention will depend on several factors, such as patient's weight, age and sex and will be easily determined by the practitioner on the basis of pharmaco-dynamics, pharmacokinetics and toxicological characteristics of the polypeptide. In general, however, said dosage will range from about 50 to about 500 mg, once to three time a day.

### REFERENCES

1. Sirtori CR. et al., Curr Atheroscler Rep. 2001; 3: 47-53.
2. Lovati MR. et al., J Nutr. 1992; 122: 1971-8.
3. Manzoni C. et al., J Nutr. 2003; 133: 2149-55.
4. Duranti M. et al., J Nutr. 2004; 134: 1334-39.
5. Thanh, VH. et al., Biochim Biophys Acta. 1976; 439: 326-38.
6. Maruyama N. et al., J Agric Food Chem. 1999; 47: 5278-84.
7. Sirtori CR. et al., Nutr Metab Cardiovasc Dis. 1998; 8: 334-40.
8. Lovati MR. et al., J Agric Food Chem. 1998; 46: 2474-80.
9. Lovati MR. et al., J Nutr. 2000; 130: 2543-2549.
10. Lovati MR. et al., Faseb J. 2006; LB 391: 86.
11. Laemmli UK. Nature. 1970; 227: 660-5.
12. Havel RY. et al., J Clin Invest. 1955; 34: 1345-53.
13. Bilheimer DW. et al.,. Biochim. Biophys. Acta 1972; 260: 212-21.
14. Sirtori CR. et al., Brit J Nutr. 2007; 97: 816-22.
15. Lovati MR. et al., Nutr Metab Cardiovasc Dis. 1991; 1: 18-24.
16. Lovati MR. et al., J Clin Invest. 1987; 80: 1498-502.
17. Kitts DD. et al., Curr Pharm Des. 2003; 9: 1309-23.
18. Wan XS. et al., Nutrition and Cancer. 2002; 43:167-73.
19. Clemente A. et al., Recent Progress in Medicinal Plants. 2008; 20:397-417.
20. Matoba N. et al., FEBS Lett. 2001; 497: 50-4.
21. Cho SJ. et al., J Agric Food Chem. 2008; 56: 4372-6.
22. Castiglioni S. et al., Atherosclerosis. 2003; 171: 163-70.
23. Sirtori CR. et al., Drug Safety 2001; 24: 665-82.

## Claims

1. A truncated recombinant Soybean 7S Globulin α' Subunit comprising the N-terminal side of the natural α' subunit.

2. The truncated recombinant α' subunit according to claim 1, consisting of 216 amino acid residues from said N-terminal side.

3. The truncated recombinant α' subunit according to claim 1, having SEQ ID#1.

4. A process for the preparation of the truncated recombinant Soybean 7S Globulin α' Subunit of claims 1-3, comprising cloning, expression in *Pichia pastoris* and purification of the recombinant polypeptide.

5. Compositions comprising as the active ingredient a truncated recombinant α' subunit of the soybean 7S globulin of claims 1-3 in admixture with a suitable carrier.
